**Europäisches Patentamt**

**European Patent Office**

**Office européen des brevets**

(19)

(11) Veröffentlichungsnummer : **0 245 802 B1**

(12) # EUROPÄISCHE PATENTSCHRIFT

(45) Veröffentlichungstag der Patentschrift :
**18.09.91 Patentblatt 91/38**

(51) Int. Cl.$^5$ : **G01N 33/86, C12Q 1/56**

(21) Anmeldenummer : **87106742.7**

(22) Anmeldetag : **08.05.87**

(54) **Analyseelement zur Bestimmung von Gerinnungsparametern.**

(30) Priorität : **16.05.86 DE 3616496**

(43) Veröffentlichungstag der Anmeldung :
**19.11.87 Patentblatt 87/47**

(45) Bekanntmachung des Hinweises auf die
Patenterteilung :
**18.09.91 Patentblatt 91/38**

(84) Benannte Vertragsstaaten :
**AT BE CH DE ES FR GB GR IT LI LU NL SE**

(56) Entgegenhaltungen :
**EP-A- 0 006 192**
**EP-A- 0 045 476**
**EP-A- 0 111 836**
**EP-A- 0 158 254**
**EP-A- 0 182 373**
**DE-A- 2 316 430**

(56) Entgegenhaltungen :
**ANALYTICAL CHEMISTRY, Band 55, Nr. 4,**
**April 1983, Seiten 498A–514A, Easton, PA, US;**
**B. WALTER "Dry reagent chemistries"**
**Seminars in Thrombosis and Hemostasis, vol**
**9, Nos 3 & 4 (1983), p172/173**
**Clin. Chem. vol 29 (1983) 225-236**
**Römpps Chemie Lexicon, 'Agarose' (p86/87),**
**'Gelatine' (p1426)**

(73) Patentinhaber : **BOEHRINGER MANNHEIM**
**GMBH**
**Sandhofer Strasse 116**
**W-6800 Mannheim 31 (DE)**

(72) Erfinder : **Bartl, Knut, Dr.rer.nat.**
**Am Westend 6**
**W-8121 Wilzhofen (DE)**
Erfinder : **Lill, Helmut, Dr.rer.nat.**
**Zugspitzstr. 24**
**W-8121 Wielenbach (DE)**
Erfinder : **Wielinger, Hans, Dr.phil.**
**Im Langgewann 7**
**W-6940 Weinheim (DE)**

EP 0 245 802 B1

## Beschreibung

Die Erfindung betrifft ein Analyseelement zur Bestimmung von Gerinnungsparametern nach dem Oberbegriff des Anspruchs 1.

Ein derartiges Analyseelement ist in der älteren, jedoch nicht vorpublizierten, deutschen Patentanmeldung DE-A- 35 16 579 beschrieben. Auf diese Anmeldung wird vollinhaltlich Bezug genommen. Dort wird als Trägermaterial bevorzugt Papier oder ein Vlies eingesetzt. In einer anderen Ausführungsform des dort beschriebenen Analyseelements befindet sich das Substrat und/oder der Cofaktor in einem wasserlöslichen, filmbildenden Polymeren. Der reagentienhaltige Film wird auf eine Basisfolie aufgebracht.

Will man mit dem in der zitierten Patentanmeldung beschriebenen Analyseelement beispielweise die Einphasengerinnungszeit nach Quick bestimmen, so enthält es Thromboplastin als Cofaktor (gelegentlich auch als Aktivator bezeichnet), Calciumionen und ein Substrat für Thrombin (eine Protease des Blutgerinnungssystems). Wird dieses Analyseelement mit Plasma in Kontakt gebracht, indem beispielsweise ein Tropfen Plasma aufgebracht wird, so wird durch die Anwesenheit des Thromboplastin und der Calciumionen die Gerinnungskaskade gestartet. Das beim Ablauf der Gerinnungskaskade entstehende Thrombin spaltet das Substrat. Das Substrat ist so ausgewählt, daß es nach der Spaltung entweder unmittelbar nachweisbar ist oder eine Nachweisreaktion auslöst. Im allgemeinen wird eine Farbbildung verwendet. Das Substrat ist in diesem Fall entweder selbst farbbildend oder das Spaltprodukt löst indirekt eine Farbbildung über eine Folgereaktion aus. Alternativ können für die Nachweisreaktionen beispielsweise auch solche Substrate verwendet werden, die fluoreszierende Farbstoffe erzeugen. Der Typ der Nachweisreaktion ist für die vorliegende Erfindung unwesentlich.

Das in der zitierten Patentanmeldung beschriebene Verfahren bedeutet einen erheblichen Fortschritt, weil erstmalig ein Analyseelement (das gelegentlich auch als Trockentest oder Testträger bezeichnet wird) zur Bestimmung von Parametern des Blutgerinnungssystems zur Verfügung gestellt wird. Es läßt jedoch noch bezüglich der Präzision der erhaltenen Meßergebnisse zu wünschen übrig. Im Rahmen der vorliegenden Erfindung wurde erkannt, daß die bei Analyseelementen üblichen Papiere oder Vliese den Ablauf der Gerinnungskaskade in vielen Fällen so beeinflussen, daß das Meßergebnis nicht den aus medizinischer Sicht wünschenswerten Grad von Genauigkeit erreicht.

Werden die Reagentien in einen wasserlöslichen Film inkorporiert, so wird eine Beeinflussung der Gerinnungskaskade im vorerwähnten Sinn bei geeigneter Wahl der Filmbildner zwar vermieden, dennoch läßt die Präzision der Messung zu wünschen übrig. Dies läßt sich, wie ebenfalls im Rahmen der vorliegenden Erfindung erkannt wurde, darauf zurückführen, daß die Filmschicht die Reagenzien verhältnismäßig langsam freisetzt. Für den Gerinnungstest müssen die Reagentien aber möglichst kurzfristig freigesetzt werden, weil die Gerinnungseigenschaften der Probe über eine Zeitmessung ermittelt werden. Der Anfangspunkt dieses Zeitraums wird durch eine verzögerte Freisetzung der Reagentien unpräzise.

Aufgabe der vorliegenden Erfindung ist es daher, ein Analyseelement für Gerinnungstests zur Verfügung zu stellen, das ebenso leicht zu handhaben ist, wie das in der zitierten Patentanmeldung beschriebene, jedoch eine verbesserte Präzision aufweist.

Die Aufgabe wird durch das in den Patentansprüchen definierte Analyseelement gelöst. Das erfindungsgemäße Analyseelement zur Bestimmung von Gerinnungsparametern des Blutes mit Hilfe von einem nachweisbaren oder eine Nachweisreaktion auslösenden Substrat einer Protease des Blutgerinnungssystems und mindestens einem Faktor und/oder Cofaktor des Blutgerinnungssystems und einer Puffersubstanz, ist dadurch gekennzeichnet, daß der Faktor und/oder Cofaktor zusammen mit einem wasserlöslichen, nichtionogenen Polymer, das den Ablauf der Gerinnungskaskade nicht verfälschend beeinflußt, auf einer offenen, flächigen, aus Fäden bestehenden Struktur, die aus einem Material besteht, das keinen störenden Einfluß auf den Ablauf der Gerinnungskaskade hat, so imprägniert ist, daß das Polymer die Struktur allseitig umgibt.

Als offene flächige Verbundstruktur ist jede Struktur zu verstehen, die flächig ausgedehnt ist, d. h. sie hat eine geringe Dicke (von bevorzugt weniger als 0,5 mm, besonders bevorzugt weniger als 0,25 mm) in Relation zu ihrer Flächenausdehnung. Die Flächenausdehnung ist durch die Testfeldfläche innerhalb des Analyseelements bestimmt. Mit "offen" ist gemeint, daß die Struktur geeignet sein soll, einer wässrigen Flüssigkeit, wie beispielsweise eine Plasmaprobe, ein schnelles Eindringen zu ermöglichen, wobei die Struktur eine möglichst große Oberfläche haben soll, die in Kontakt mit der Probe gebracht werden kann. Besonders bevorzugt ist ein Gewebe, ein Gewirke oder ein Netz, das aus Fäden besteht. Insbesondere sind monofile Fäden geeignet.

Als Material zur Herstellung der flächigen Verbundstruktur ist im Prinzip jedes Material geeignet, das keinen störenden Einfluß auf den Ablauf der Gerinnungskaskade hat und aus dem sich fertigungstechnisch eine Struktur der beschriebenen Art herstellen läßt. Außerdem muß das Material so beschaffen sein, daß das nichtionogene Polymer mit den Reagentien darauf haftet. Besonders bevorzugte Materialien sind Polyamid, Polyester und Mischgewebe aus diesen Fasern. Die Fadenzahl pro cm liegt bevorzugt über 20.

Die Frage, ob das für die Verbundstruktur gewählte Material den Ablauf der exogenen Gerinnungskaskade beeinflußt, läßt sich wie folgt testen: 40 mg des Materials werden mit 300 µl Citratplasma 60 sec bei 37 °C inkubiert. Danach wird das Plasma abzentrifugiert, die Gerinnbarkeit des Plasmas darf sich nicht statistisch signifikant geändert haben.

Das nichtionogene Polymer muß die Eigenschaft haben, daß es sich bei den bei der Anwendung üblichen Temperaturen hinreichend schnell im wäßrigen Medium löst. Es wurde gefunden, daß auch Materialien, die sich bei größerer Schichtstärke erst bei oberhalb der Raumtemperatur liegenden Temperaturen in Wasser ausreichend schnell lösen, noch geeignet sind, weil sie auf der erfindungsgemäßen Verbundstruktur in dünner Schicht vorliegen.

Weiterhin muß das nichtionogene Polymer die Eigenschaft haben, auf der flächigen Verbundstruktur eine haftende, dünne Schicht zu bilden. Im Gegensatz zu einer Beschichtung, wie sie beispielsweise bei der Herstellung regenabweisender Kleidung üblich ist, bei der ein Polymermaterial auf einer Gewebeschicht dergestalt aufgebracht wird, daß die Polymerschicht im wesentlichen auf einer Seite der Gewebestruktur bleibt und deren Öffnungen überspannt, wird die erfindungsgemäße Verbundstruktur mit dem nichtionogenen Polymer imprägniert, d. h. das Polymer umgibt die Verbundstruktur allseitig.

Geeignete Polymere sind beispielsweise nichtionogene Cellulose-Derivate, Polyvinylpyrrolidone und Polyxanthane.

Als besonders geeignet haben sich Polyethylenoxide, Polyacrylamide sowie teilweise oder vollständig zu Polyvinylalkohol verseifte Polyvinylacetate erwiesen. Innerhalb der letztgenannten Gruppe sind die teilweise verseiften Polyvinylacetate bevorzugt. Besonders bevorzugt sind Polyvinylacetate mit einem Verseifungsgrad zwischen 70 und 93 Mol.-%. Alle diese Polymere haben überraschenderweise den zusätzlichen Vorteil, daß sie das Thromboplastin stabilisieren. Auf diese Weise läßt sich ein Quick-Test herstellen, der sich durch ein besonders gutes Lagerverhalten auszeichnet.

Gegebenenfalls können auch Gemische geeigneter nichtionogener Polymere zur Anwendung kommen.

Zur Herstellung der erfindungsgemäßen Reagenzträger wird zunächst eine Lösung des Faktors und/oder des Cofaktors und des nichtionogenen wasserlöslichen Polymeres hergestellt. Gegebenenfalls können noch andere Bestandteile, beispielsweise Weichmacher, zugegeben werden. Mit dieser Lösung wird die flächige Verbundstruktur bevorzugt durch Eintauchen, unter Umständen auch durch Besprühen imprägniert. Die geeignete Viskosität der Imprägnierungslösung kann nicht generell festgelegt werden, sondern ist für jedes nichtionogene Polymer bzw. Polymergemisch unter produktionstechnischen Gesichtspunkten auf das jeweilige Gewebe abzustimmen. Gut bewährt haben sich Viskositäten zwischen 50 und 250 mPasec.

Das als Indikator wirkende Substrat kann zusammen mit dem Faktor und/oder dem Cofaktor und dem nichtionogenen wasserlöslichen Polymeren auf der flächigen Verbundstruktur imprägniert werden. Unter Umständen kann es jedoch problematisch sein, den Faktor und/oder Cofaktor zusammen mit dem Substrat zu verarbeiten. In diesem Falle hat es sich als zweckmäßig erwiesen, das Substrat auf einem separaten Trägermaterial unterzubringen. Für diesen Zweck kommen verschiedene bekannte Trägermaterialien in Betracht. Besonders bevorzugt wird jedoch auch in diesem Fall eine offene, flächige Verbundstruktur als Trägermaterial verwendet, welche analog dem vorbeschriebenen Verfahren mit einer Lösung imprägniert wird, welche neben dem Substrat ein nichtionogenes wasserlösliches Polymer enthält. Dadurch ergibt sich ein besonders gleichmäßiges Auflösungsverhalten der beiden Trägermaterialschichten, die einerseits den Faktor und/oder den Cofaktor und andererseits das als Indikator dienende Substrat enthalten.

Gemäß einer weiteren bevorzugten Ausführungsform wird die offene, flächige Verbundstruktur bzw. soweit mehrere derartige Strukturen verwendet werden, mindestens eine davon gleichzeitig mit dem bei Gerinnungstests im allgemeinen zur Recalcifizierung des Plasmas erforderlichen Calciumionen imprägniert.

Puffersubstanzen können ebenfalls zusammen mit dem Faktor und/oder Cofaktor imprägniert werden, sie können jedoch auch auf einem separaten Trägermaterial des Analyseelements vorhanden sein.

Die Erfindung wird im folgenden anhand der Figur und anhand von Ausführungsbeispielen näher erläutert.

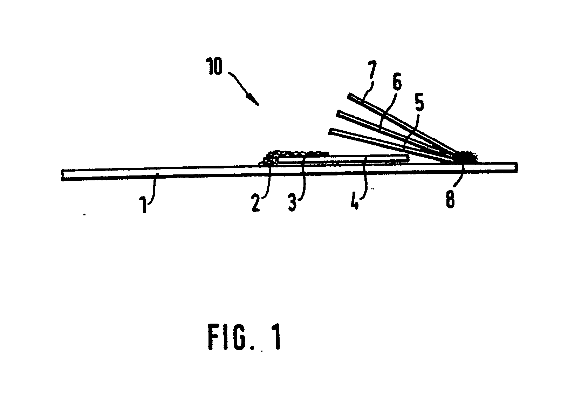

Fig. 1 zeigt ein erfindungsgemäßes Analyseelement schematisch im Querschnitt.

Man erkennt als tragenden Bestandteil des allgemein mit 10 bezeichneten Analyseelements eine Trägerfolie 1. Im dargestellten Ausführungsbeispiel hat sie eine längliche Form wie bei einem Teststreifen. Die Erfindung ist jedoch auch für andere Formen von Analyseelementen geeignet, die beispielsweise eine quadratische Form ähnlich der von fotografischen Diapositiven haben.

Auf der Trägerfolie ist mit einem Streifen Schmelzkleber 2 ein Erythrozytenabtrenn- und Plasmatransportvlies befestigt. Besonders eignet sich ein Vlies aus einen gerinnungsneutralen Glasfasermaterial, wie es in den deutschen Patentanmeldungen 35 23 969 und 36 10 429 (EP-A- 02 39 002) beschrieben ist. Das im folgenden kurz als Transportvlies bezeichnete Vlies 4 is teilweise mit einem ebenfalls mit dem Kleber 2 befestigten Abdecknetz 3 überdeckt. Hierfür ist ein Nylonnetz geeignet.

Drei weitere flächenförmige Bestandteile des Analyseelements 10 sind mit einem Streifen Schmelzkleber

3

dergestalt auf der Trägerfolie 1 befestigt, daß sie das Transportvlies 4 teilweise überlappen. Da sie nur an einer ihrer Kanten aneinander und an der Trägerfolie 1 befestigt sind, haben sie über ihre verbleibende Fläche einen Abstand voneinander, solange sie nicht durch äußeren Druck aufeinander gedrückt werden. Näheres zu dieser Art des Testaufbaus ist beispielsweise der europäischen Patentanmeldung mit der Publikationsnummer 45 476 zu entnehmen.

Die unterste Schicht 5 kann ein Oxidationsmittelträger sein, der im folgenden auch als Oxidationsmatrix bezeichnet wird. Darüber befindet sich der erfingungsgemäße Reagenzträger 6 in Form der imprägnierten offenen Verbundstruktur. Als oberste Schicht ist eine Abdeckfolie 7 vorgesehen.

Bevorzugt wird für die Schicht 6 ein Gewebe verwendet, das mit einer Lösung imprägniert ist, in der die Filmbildner, Puffer, Thromboplastin oder ein anderes Startreagenz für den Start der exogenen oder endogenen Gerinnungskaskade sowie ein zugleich als Indikator wirkendes Substrat für eine der beim Ablauf der Blutgerinnung entstehenden Proteasen, wie z. B. Faktor II a, Faktor X a usw., eventuell noch ein Kuppler, sowie Calcium-Ionen und eventuell noch ein Weichmacher für den Filmbildner im Wasser gelöst sind. Als Puffersubstanzen haben sich Tris und Verbindungen aus der Reihe der Good-Puffer bewährt. Als Substrate können alle Proteasesubstrate genommen werden, die aufgrund ihrer Aminosäuresequenz ausreichend spezifisch sind, wie z. B. Tos-gly-pro-arg-4-nitroanilid-acetat, oder

Tos-gly-pro-arg-p-phenylendiamin-acetat und N-Methylanthranilsäure als Kuppler, andere Kuppler, wie N-(4-Fluorphenyl)-N-methylaminomethanphosphonsäure usw., können ebenfalls eingesetzt werden.

Bei Indikatoren, die nach Spaltung durch die Protease über eine angeschlossene oxidative Kupplung eine Farbe geben, ist es notwendig, in der Schicht 5 ein Oxidationsmittel, wie z. B. $K_3[Fe(CN)_6]$ vorzusehen. Gegebenenfalls können in der Schicht 5 auch Calciumionen untergebracht werden. Bevorzugt wird für die Schicht 5 ebenfalls ein dünnes Gewebe verwendet, das imprägniert wurde. Soweit ein Indikator verwendet wird, bei dem keine Verstärkungsreaktion über eine oxidative Kupplung erforderlich ist, entfällt die Oxidationsmatrix 5.

Die Schicht 5 kann bei der oben erwähnten alternativen Ausführungsform mit separater Unterbringung des Substrats auch eine flächige Verbundstruktur sein, auf die das Substrat getrennt von dem Faktor und/oder Cofaktor imprägniert ist.

Nähere Einzelheiten über die chemische Zusammensetzung verschiedener Gerinnungstests, die auch für die vorliegende Erfindung geeignet sind, sind der eingangs zitierten deutschen Patentanmeldung 35 16 579 zu entnehmen.

In den folgenden Beispielen werden die Ergebnisse, wie sie mit Reagenzfilmen auf Folie gemäß der zitierten deutschen Patentanmeldung erzielt werden, verglichen mit Ergebnissen mit erfindungsgemäß imprägnierten Geweben.

**Herstellung eines Analyseelements zur Bestimmung der Einphasengerinnungszeit nach Quick**

**Herstellung von Reagenzfilmen (Reagenzmatrix) auf Folie**

Auf Polycarbonatfolien der Dicke 200 μm werden mit einer Naßfilmdicke von 100 μm Filme in der Tab. 1 aufgeführten Zusammensetzungen aufgerakelt und bei 45 °C getrocknet. Nach dem Trocknen werden die beschichteten Folienbahnen auf eine Breite von 15 mm geschnitten.

**Herstellung von imprägnierten Geweben (erfindungsgemäß)**

Polyamidgewebe (Typ 75 HC der Firma Züricher Beuteltuchfabrik, Schweiz), werden mit Imprägnierlösungen der in Tab. 1 aufgeführten Zusammensetzungen imprägniert und bei 45 °C getrocknet. Nach dem Trocknen werden die imprägnierten Gewebebahnen auf eine Breite von 15 mm geschnitten.

**Herstellung der Oxidationsmatrix**

Ein Nylonnetz (Type NY 20 HC Super der Firma Züricher Beuteltuchfabrik, Schweiz), wird mit einer wässrigen Lösung von 50 mmol $K_3[Fe(CN)_6]$/l, 50 mmol Calciumchlorid/l und 0,2 g/l Polyacrylamid (Rohagit 700 der Firma Röhm) imprägniert und bei 45 °C getrocknet. Nach dem Trocknen werden von dem imprägnierten Netz 15 mm breite Bahnen geschnitten.

**Herstellung der Beschichtungs- bzw. Imprägniermassen**

In 1 Liter destilliertes Wasser werden Hepes 100 mmol; Tos-Gly-Pro-Arg-p-phenylendiamin 1 mmol; N-(4-fluorphenyl)-N-methylaminomethanphosphonsäure 15 mmol; 2,5 g Thromboplastin und die Polymere entsprechend den in Tabelle 1 gemachten Angaben gelöst. Diese Lösungen werden mit Natronlauge auf einen pH von 7,5 eingestellt.

## Tabelle 1:

| Polymere | Menge in der Beschichtungsmasse | Menge in der Imprägnierlösung |
|---|---|---|
| 1. Polyvinylalkohol Mowiol (26/88, Hoechst) | 70 g | 40 g |
| 2. Polyvinylalkohol Mowiol (18/88, Hoechst) | 75 g | 50 g |
| 3. Polyethylenoxid (Polyox 301, Union Carbide) | 10 g | 1 g |
| Polyacrylamid (Rohagit 700, Röhm) | 5 g | 2,5 g |
| 4. Polyvinylalkohol Mowiol 18/88, Hoechst) | 60 g | 40 g |
| Polyacrylamid (Rohagit 700, Röhm) | 10 g | 5 g |

**Herstellen des Analyseelements**

Auf eine 100 mm breite Polystyrolfolie (1 in Abb. 1) wird ein Glasfaservlies (4 in Abb. 1) gemäß der deutschen Patentanmeldung 35 23 969 von einem Flächengewicht von 30 - 40 g/m² in einer Breite von 15 mm gelegt, mit einem Nylonnetz (3 in Abb. 1) teilweise abgedeckt und an einem Ende mit einem Kleber (2 in Abb. 1) fixiert. Am freien Ende des Glasfaservlies werden die Oxidations- (5 in Abb. 1) und Reagenzmatrix (6 in Abb. 1) übereinandergelegt und mit einer 200 µm dicken transparenten Polycarbonatfolie (7 in Abb. 1) von 15 mm Breite abgedeckt und mit einem Kleber 8 (2 in Abb. 1) festgeklebt. Die so hergestellten Bänder werden zu 6 mm breiten Streifen geschnitten.

Der Aufbau des Analyseelements mit einem Reagenzfilm auf Folie entspricht im wesentlichen dem beschriebenen Aufbau gemäß Abb. 1. Er unterscheidet sich jedoch dadurch, daß statt der Schichten 6 und 7 eine einzige Schicht vorgesehen ist. Diese besteht aus einer durchsichtigen Folie, die mit dem Film beschichtet ist. Sie ist so angeordnet, daß die Folie in der Figur nach oben weist.

**Vergleich der mit Filmbeschichtung auf Folie und dem erfindungsgemäßen Verfahren zu erreichenden Präzisionen bei der Bestimmung der Einphasengerinnungszeit nach Quick**

Dazu wird wie folgt vorgegangen:

5

Auf die oben beschriebenen Teststreifen werden 32 µl Poolplasma von einem Quickwert von 100 % und einem durch Verdünnung mit physiologischer Kochsalzlösung hergestelltem Plasma von 12,5 % pipettiert. Der mit den Plasmaproben beschickte Reagenzträger wird im Remissionsphotometer "Reflotron", Boehringer Mannheim vermessen. Es wird die Zeit gemessen, die vergeht, bis eine Remissionsabnahme von 10 % stattgefunden hat, d. h. die Zeit, bis die exogene Gerinnungskaskade soweit abgelaufen ist, daß eine bestimmte Menge an Thrombin (Faktor II a) entstanden ist.

Bei den Meßreihen wurden jeweils 25 Bestinmungen je Testvariante und % Quick durchgeführt.

Die Meßergenisse sind in Tabelle 2 dargestellt.

Die vier Zeilen der Tabelle beziehen sich auf die vier verschiedenen Polymere bzw. Polymergemische der Tabelle 1. In den ersten beiden Spalten sind die Meßergebnisse für den zum Vergleich herangezogenen Film auf Folie dargestellt. Die letzten beiden Spalten zeigen die Meßergebnisse für die erfindungsgemäße Ausführungsform.

Für jede der Ausführungsformen sind die gemessenen Zeiten für das 100 %-Plasma und für das 12,5 %-Plasma angegeben. Wesentlich für die Beurteilung der Erfindung sind insbesondere die ebenfalls angegebenen Variationskoeffizienten (VK) in Prozent.

Die in der Tabelle wiedergegebenen Meßergebnisse zeigen deutlich, daß mit der erfingungsgemäßen Ausführung statistisch signifikant bessere Variationskoeffizienten erhalten werden. Im Bereich von niedrigen Quickprozenten, im wichtigen therapeutischen Bereich, tritt die verbesserte Präzision besonders deutlich hervor.

Tabelle 2

| Polymer | Filmbeschichtung | | | | erfindungsgemäße Ausführung | | | |
|---|---|---|---|---|---|---|---|---|
| | 100 % Quick | | 12,5 % Quick | | 100 % Quick | | 12,5 % Quick | |
| | sec | VK in % | sec | VK in % | sec | VK in % | sec | VK in % |
| 1 | 42,4 | 3,4 | 118,0 | 9,4 | 41,6 | 2,6 | 66,1 | 3,1 |
| 2 | 42,9 | 2,1 | 123,2 | 10,3 | 39,6 | 1,4 | 67,3 | 3,2 |
| 3 | 44,2 | 4,9 | 83,2 | 5,2 | 35,1 | 1,7 | 83,3 | 2,3 |
| 4 | 44,9 | 4,1 | 110,7 | 7,1 | 38,1 | 1,5 | 70,2 | 2,2 |

EP 0 245 802 B1

EP 0 245 802 B1

## Patentansprüche

1. Analyseelement zur Bestimmung von Gerinnungsparametern des Blutes mit Hilfe von einem nachweisbaren oder eine Nachweisreaktion auslösenden Substrat einer Protease des Blutgerinnungssystems und mindestens einem Faktor und/oder cofaktor des Blutgerinnungssystems und einer Puffersubstanz, dadurch gekennzeichnet, daß der Faktor und/oder cofaktor zusammen mit einem wasserlöslichen, nichtionogenen Polymer, das den Ablauf der Gerinnungskaskade nicht verfälschend beeinflußt, auf einer offenen, flächigen, aus Fäden bestehenden Struktur, die aus einem Material besteht, das keinen störenden Einfluß auf den Ablauf der Gerinnungskaskade hat, so imprägniert ist, daß das Polymer die Struktur allseitig umgibt.

2. Analyselement nach Anspruch 1, dadurch gekennzeichnet, daß das Substrat zusammen mit dem Faktor und/oder Cofaktor, und dem wasserlöslichen nichtionogenen Polymer auf der offenen, flächigen, aus Fäden bestehenden Struktur imprägniert ist.

3. Analyseelement nach Anspruch 1, dadurch gekennzeichnet, daß das Substrat zusammen mit einem wasserlöslichen nichtionogenen Polymer auf eine zweite offene, flächige, aus Fäden bestehende Struktur imprägniert ist, welche von der den Faktor und/oder Cofaktor tragenden Struktur getrennt ist.

4. Analyseelement nach einem der Ansprüche 1 bis 3, dadurch gekennzeichnet, daß die flächige, offene, aus Fäden bestehende Struktur zusätzlich mit Calciumionen imprägniert ist.

5. Analyselement nach Anspruch 1, dadurch gekennzeichnet, daß die flächige, aus Fäden bestehende Struktur ein Gewebe, Gewirke oder Netz ist.

6. Analyseelement nach Anspruch 5, dadurch gekennzeichnet, daß das Gewebe, Gewirke oder Netz aus monophilen Fäden besteht.

7. Analyseelement nach Anspruch 5, dadurch gekennzeichnet, daß die flächige, aus Fäden bestehende Struktur eine Dicke von höchstens 0,25 mm hat und mindestens 20 Fäden pro cm aufweist.

8. Analyseelement nach Anspruch 1, dadurch gekennzeichnet, daß das nichtionogene Polymer ein Filmbildner ist, der bei Temperaturen unter 50 °C in Wasser löslich ist.

9. Analyseelement nach Anspruch 1, bei dem als Cofaktor Thromboplastin verwendet wird, dadurch gekennzeichnet, daß das wasserlösliche, nichtionogene Polymer ein Polyethylenoxid, ein Polyacrylamid oder ein Polyvinylalkohol ist.

## Revendications

1. Elément d'analyse pour la détermination des paramètres de coagulation du sang, à l'aide d'un substrat, détectable ou initiant une réaction de détection, d'une protéase du système de coagulation du sang et d'au moins un facteur et/ou cofacteur du système de coagulation du sang, et d'une substance tampon, caractérisé en ce qu'une structure ouverte, plate, constituée de fibres, formée d'une substance qui n'a pas d'action pouvant perturber le déroulement des étapes de la coagulation, est imprégnée à la fois par le facteur et/ou par le cofacteur, par un polymère hydrosoluble, non ionogène, qui n'agit pas de façon à fausser le déroulement des étapes de la coagulation, de façon que le polymère entoure la structure de toutes parts.

2. Elément d'analyse selon la revendication 1, caractérisé en ce que la structure ouverte, plate, constituée de fibres, est imprégnée à la fois par le substrat, par le facteur et/ou par le cofacteur, et par le polymère hydrosoluble non ionogène.

3. Elément d'analyse selon la revendication 1, caractérisé en ce qu'une deuxième structure ouverte, plate, constituée de fibres, qui est séparée de la structure portant le facteur et/ou le cofacteur, est imprégnée à la fois par le substrat et par un polymère hydrosoluble non ionogène.

4. Elément d'analyse selon l'une des revendications 1 à 3, caractérisé en ce que la structure ouverte, plate, constituée de fibres est également imprégnée d'ions de calcium.

5. Elément d'analyse selon la revendication 1, caractérisé en ce que la structure ouverte, plate, constituée de fibres est un tissu, une étoffe en mailles ou un filet.

6. Elément d'analyse selon la revendication 5, caractérisé en ce que le tissu, l'étoffe en mailles ou le filet sont constitués de fibres monofils.

7. Elément d'analyse selon la revendication 5, caractérisé en ce que la structure plate, constituée de fibres, présente une épaisseur d'au plus 0,25 mm et présente au moins 20 fibres par cm.

8. Elément d'analyse selon la revendication 1, caractérisé en ce que le polymère non ionogène est un formateur de film qui est soluble dans l'eau à des températures inférieures à 50°C.

9. Elément d'analyse selon la revendication 1, dans lequel, en tant que cofacteur, on utilise la thrombo-

8

plastine, caractérisé en ce que le polymère hydrosoluble non ionogène est un oxyde de polyéthylène, un polyacrylamide ou un poly(alcool vinylique).

## Claims

1. Analysis element for the determination of coagulation parameters of blood with the help of a detectable or a detection reaction-initiating substrate of a protease of the blood coagulation system and at least one factor and/or co-factor of the blood coagulation system and a buffer substance, characterised in that the factor and/or co-factor, together with a water-soluble, non-ionogenic polymer which does not falsely influence the course of the coagulation cascade, is so impregnated on an open, planar structure consisting of filaments which consists of a material which has no disturbing influence on the course of the coagulation cascade that the polymer surrounds the structure on all sides.

2. Analysis element according to claim 1, characterised in that the substrate is impregnated on to an open, planar structure consisting of filaments, together with the factor and/or co-factor and the water-soluble, non-ionogenic polymer.

3. Analysis element according to claim 1, characterised in that the substrate is impregnated on to a second open, planar structure consisting of filaments, together with a water-soluble, non-ionogenic polymer, which is separate from the structure carrying the factor and/or co-factor.

4. Analysis element according to one of claims 1 to 3, characterised in that the planar, open structure consisting of filaments is additionally impregnated with calcium ions.

5. Analysis element according to claim 1, characterised in that the planar structure consisting of filaments is a fabric, textile or mesh.

6. Analysis element according to claim 5, characterised in that the fabric, textile or mesh consists of monofilar filaments.

7. Analysis element according to claim 5, characterised in that the planar structure consisting of filaments has a thickness of at most 0.25 mm. and has at least 20 filaments per cm.

8. Analysis element according to claim 1, characterised in that the non-ionogenic polymer is a film former which is soluble in water at temperatures below 50°C.

9. Analysis element according to claim 1 in which thromboplastin is used as co-factor, characterised in that the water-soluble, non-ionogenic polymer is a polyethylene oxide, a polyacrylamide or a polyvinyl alcohol.

FIG. 1